# FASCICULE DE BREVET EUROPEEN

(11) **EP 1 135 389 B1**
(45) Date de publication et mention de la délivrance du brevet: **10.09.2003**
(21) Numéro de dépôt: 99973291.0
(22) Date de dépôt: 01.12.1999
(51) Int. Cl.: C07D 471/08, A61K 31/551

(54) **DERIVES DE 1,4-DIAZABICYCLO 3.2.2]NONANE, LEUR PREPARATION ET LEUR APPLICATION EN THERAPEUTIQUE**
1,4-DIAZABICYCLO(3.2.2)NONANDERIVATE, DEREN HERSTELLUNG UND DEREN VERWENDUNG IN HEILKUNDE
1,4-DIAZABICYCLO 3.2.2]NONANE DERIVATIVES, THEIR PREPARATION AND THERAPEUTIC APPLICATION

(30) Priorité: 04.12.1998 FR 9815326
(43) Date de publication de la demande: 26.09.2001
(73) Titulaire: SANOFI-SYNTHELABO, 75013 Paris (FR)
(72) Inventeur: LOCHEAD, Alistair, F-94220 Charenton (FR); JEGHAM, Samir, F-34980 Montferrier sur Lez (FR); NEDELEC, Alain, F-92700 Colombes (FR); GALLI, Frédéric, F-92420 Vaucresson (FR); JEUNESSE, Jean, F-94500 Champigny sur Marne (FR); EVEN, Luc, F-75015 Paris (FR)
(74) Mandataire: Ludwig, Jacques
(86) Numéro de dépôt international: FR9902975
(87) Numéro de publication internationale: WO00034279

(56) Documents cités:
- EP-A- 0 400 661
- WO-A-96/30372
- US-A- 5 478 939
- B.R. DE COSTA ET AL.: "Synthesis and evaluation of conformationally restricted N-[2-(3,4-dichlorophenyl)ethyl]-N-methyl-2 -(1-pyrrolidinyl)ethylamines at sigma receptors" JOURNAL OF MEDICINAL CHEMISTRY., vol. 36, no. 16, 6 août 1993 (1993-08-06), pages 2311-2320, XP002130550 AMERICAN CHEMICAL SOCIETY. WASHINGTON., US ISSN: 0022-2623 cité dans la demande

## Description

Les composés de la présente invention répondent à la formule générale (I) 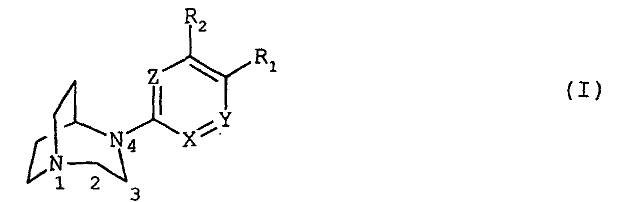 dans laquelle
l'un des symboles X, Y et Z représente un atome d'azote, un autre représente un groupe de formule C-R₃ et le troisième représente un atome d'azote ou un groupe de formule C-R₄, R₃ et R₄ représentent chacun, indépendamment l'un de l'autre, un atome d'hydrogène ou d'halogène ou un groupe trifluorométhyle, cyano, hydroxy, (C₁-C₆)alkyle ou (C₁-C₆)alcoxy,
R₁ et R₂ représentent chacun, indépendamment l'un de l'autre, un atome d'hydrogène ou d'halogène ou un groupe trifluorométhyle, cyano, hydroxy, (C₁-C₆)alkyle, (C₁-C₆)alcoxy, ou phényle éventuellement substitué par un atome d'halogène, par un ou deux groupes trifluorométhyle, par un groupe cyano, par un groupe nitro, par un groupe hydroxy, par un groupe (C₁-C₆)alkyle, par un groupe (C₁-C₆)alcoxy, par un groupe acétyle, par un groupe méthylènedioxy, par un groupe trifluorométhoxy, par un groupe méthylthio ou par un groupe phényle.

Les composés de l'invention peuvent exister à l'état de bases ou de sels d'addition à des acides.

Les composés préférés sont ceux dans la formule desquels l'hétérocycle contenant X, Y et Z est un groupe pyridin-3-yle ou pyridazin-3-yle.

La demande internationale WO 96 30372 décrit un composé de structure analogue à celle des composés de l'invention, et ayant la même propriété biologique.

Conformément à l'invention, et selon le schéma qui suit, on peut préparer les composés de formule générale (I) en faisant réagir le 1,4-diazabicyclo[3.2.2]nonane, de formule (II), avec un composé hétérocyclique de formule générale (III), dans laquelle X, Y, Z, R₁ et R₂ sont tels que définis ci-dessus et W représente un atome d'halogène.

On peut ainsi effectuer un couplage de Buchwald (*J. Org. Chem.* (1997) **62** 6066-6068) en présence d'un catalyseur au 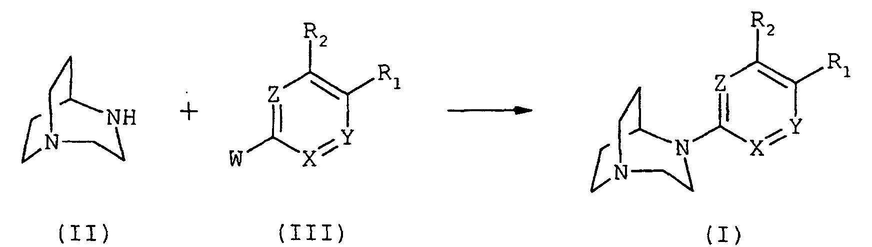 palladium tel que l'acétate de palladium, le tris(dibenzylidèneacétone)dipalladium(0), etc, d'un ligand de complexation tel que la triphénylphosphine, la tributylphosphine ou le 2,2'-bis(diphénylphosphino)-1,1'-binaphtyle, et d'une base, par exemple organique telle que le t-butoxyde de sodium, ou minérale telle que le carbonate de césium.

Lorsque X ou Z représente un atome d'azote on peut aussi effectuer une réaction de substitution nucléophile en présence d'une base forte telle que le carbonate de césium ou la triéthylamine.

Le 1,4-diazabicyclo[3.2.2]nonane est décrit dans *J. Med. Chem.* (1993) **36** 2311-2320.

Pour certains composés , les substituents R₁ et/ou R₂ ne sont pas présents dans le composé de départ de formule générale (III) ; selon leur nature, ces substituents peuvent être introduits sur le composé final de formule générale (I). Ainsi, par exemple des composés de formule générale (I) dans lesquelles R₁ et/ou R₂ représentent des groupements aryles peuvent être préparés à partir des composés correspondants, dans la formule desquels R₁ et/ou R₂ représentent des atomes de brome ou d'iode selon toutes méthodes connues, telles qu'un couplage Suzuki en présence d'un acide boronique et d'un catalyseur au palladium, par exemple le tétrakistriphénylphosphine palladium.

Les composés de formule générale (III) sont disponibles dans le commerce ou sont accessibles par des méthodes décrites dans la littérature.

Les exemples qui vont suivre illustrent la préparation de quelques composés de l'invention. Les microanalyses élémentaires, et les spectres I.R. et R.M.N. confirment les structures des composés obtenus.
Les numéros indiqués entre parenthèses dans les titres des exemples correspondent à ceux de la lère colonne du tableau 1 donné plus loin.
Dans les noms des composés, le tiret "-" fait partie du mot, et le tiret "_" ne sert que pour la coupure en fin de ligne ; il est à supprimer en l'absence de coupure, et ne doit être remplacé ni par un tiret normal ni par un espace.

### Exemple 1 (Composé N°1).

Bromhydrate de 4-pyridin-3-yl-1,4-diazabicyclo[3.2.2]nonane (2:1).

Dans un ballon tricol de 100 ml on place 1,0 g (7,92 mmoles) de 1,4-diazabicyclo[3.2.2]nonane, 5,0 g (31,7 mmoles) de 3-bromopyridine, 88,9 mg (0,396 mmole) d'acétate de palladium(II), 247 mg (0,396 mmole) de 2,2'-bis(diphé_ nylphosphino)-1,1'-binaphtyle et 3,61 g (11,1 mmoles) de carbonate de césium en suspension dans 50 ml de tétrahydro_ furane, et on chauffe le mélange au reflux pendant 72 h.
On le laisse refroidir, on le filtre, on évapore le filtrat sous pression réduite et on purifie le résidu par chromatographie sur colonne de gel de silice en éluant avec un mélange 95/5/0,5 de chloroforme, méthanol et ammoniaque.
On obtient 0,83 g d'une huile orangée qu'on met en suspension dans 15 ml d'éthanol et on ajoute 1,45 ml d'une solution d'acide bromhydrique à 37% dans l'acide acétique. On isole finalement 1,02 g de dibromhydrate.
Point de fusion : 295-305°C.

### Exemple 2 (Composé N°6).

Bromhydrate de 4-(6-chloropyridazin-3-yl)-1,4-diazabicyclo_ [3.2.2]nonane (2:1).

Dans un ballon tricol de 50 ml on introduit 0,88 g (7,0 mmoles) de 1,4-diazabicyclo[3.2.2]nonane et 0,52 g (3,5 mmoles) de 3,6-dichloropyridazine dans 25 ml de toluène, et on chauffe la solution au reflux pendant 24 h.
On évapore le solvant sous pression réduite, on reprend le résidu avec un mélange 90/10 de dichlorométhane et de méthanol contenant quelques gouttes d'ammoniaque, et on élue la solution sur gel de silice avec le même mélange de solvants.
On obtient 0,58 g de composé sous forme de base, qu'on transforme en dibromhydrate par addition d'une solution d'acide bromhydrique à 37% dans l'acide acétique.
Après recristallisation dans un mélange 50/50 de propan-2-ol et de méthanol on isole finalement 0,7 g de dibromhydrate.
Point de fusion : 291-293°C.

### Exemple 3 (Composé N°8).

Bromhydrate de 4-(6-phénylpyridin-3-yl)-1,4-diazabicyclo_ [3.2.2 ]nonane (2:1).

### 3.1. 3-Bromo-6-phénylpyridine.

Dans un ballon tricol de 250 ml on introduit 10 g (42,2 mmoles) de 2,5-dibromopyridine, 5,2 g (42,2 mmoles) d'acide phénylboronique et 30 ml de benzène, on ajoute 1,5 g (1,3 mmole) de tétrakis(triphénylphosphino)palladium, 30 ml de benzène et 30 ml d'une solution aqueuse de carbonate de sodium à 2M et 1,4 ml d'éthanol et on chauffe le mélange à reflux pendant 17 h.
On refroidit le milieu réactionnel, on le filtre, on décante la phase organique, on évapore le solvant sous pression réduite et on purifie le résidu par chromatographie sur gel de silice en éluant avec un mélange 70/30 d'heptane et de dichlorométhane.
On obtient 8,6 g de produit brut que l'on recristallise dans 7 ml d'éthanol. On obtient ainsi 5,6 g de produit pur sous forme de solide blanc.
Point de fusion : 69-72°C.

### 3.2 Bromhydrate de 4-(6-phénylpyridin-3-yl)-1,4-diazabi_ cyclo[3.2.2 ]nonane (2:1).

Dans un ballon tricol on charge, sous atmosphère d'azote, 0,631 g (5 mmoles) de 1,4-diazabicyclo[3.2.2 ]nonane, 1,4 g (6 mmoles) de 3-bromo-6-phénylpyridine, 2,28 g (7 mmoles) de carbonate de césium, 45 mg (0,2 mmole) d'acétate de palladium(II) et 125 mg (0,2 mmole) de 2,2'-bis(diphényl_ phosphino)-1,1'-binaphtyle en suspension dans 40 ml de tétrahydrofurane, et on porte le mélange à reflux pendant 48 h.
On refroidit le milieu réactionnel, on le filtre et on évapore le solvant sous pression réduite. On purifie le résidu obtenu par chromatographie sur gel de silice en éluant avec un mélange 96/4/0,4 de chloroforme, méthanol et ammoniaque. On obtient 0,47 g de produit brut que l'on reprend dans de l'éther. On élimine un insoluble par filtration et on concentre le filtrat sous pression réduite pour obtenir 0,42 g de produit.
On le met en suspension dans 10 ml d'éthanol et on traite la solution obtenue avec 0,6 ml d'une solution d'acide bromhydrique dans l'acide acétique à 5M. Après 1 h on recueille le précipité, on le rince à l'éthanol, puis à l'éther. On obtient ainsi 0,519 g de produit sous forme de solide blanc.
Point de fusion : 290-297 °C.

### Exemple 4 (Composé N°9).

Bromhydrate de 4-(5-bromopyridin-3-yl)-1,4-diazabicyclo_ [3.2.2 ]nonane (2:1).

Dans un ballon tricol de 250 ml, on ajoute à une suspension de 71 mg (0,32 mmole) d'acétate de palladium(II), 3,6 g (11,09 mmoles) de carbonate de césium et 0,197 mg (0,32 mmole) de 2,2'-bis(diphénylphosphino)-1,1'-binaphtyle dans 45 ml de tétrahydrofurane, une solution contenant 1,0 g (7,92 mmoles) de 1,4-diazabicyclo[3.2.2 ]nonane et 2,82 g (11,89 mmoles)de 3,5-dibromopyridine dans 20 ml de tétrahydrofurane. On chauffe le milieu réactionnel pendant 22 h.
On refroidit la suspension et on dilue avec 65 ml de chloroforme. On élimine un précipité par filtration et on concentre le filtrat sous pression réduite pour obtenir 4,4 g de solide brun foncé. On purifie le produit par chromatographie sur gel de silice en éluant avec un mélange 95/5/0,5 de chloroforme, méthanol et ammoniaque. On obtient 1,25 g de produit pur que l'on dissout dans 10 ml d'éthanol et traite avec 1,6 ml d'une solution d'acide bromhydrique dans l'acide acétique à 5,7 M à 0°C. On obtient 1,79 g de produit que l'on recristallise dans 20 ml d'un mélange 86/14 d'éthanol/eau. On obtient ainsi 1,14 g de produit sous forme de solide jaune pâle.
Point de fusion : 266-276 °C.

### Exemple 5 (Composé N°10).

### 4-(5-Phénylpyridin-3-yl)-1,4-diazabicyclo[3.2.2]nonane.

On introduit 0,87 g (3,1 mmoles) de 4-(5-bromopyridin-3-yl)-1,4-diazabicyclo[3.2.2 ]nonane dans un ballon tricol de 50 ml, on ajoute 0,39 g (3,15 mmole) d'acide phénylboro_ nique, 0,11 g (0,095 mmole) de tétrakis(triphénylphos_ phino)palladium, 3,3 ml d'une solution aqueuse de carbonate de sodium à 2 M, 6,5 ml de benzène et 0,15 ml d'éthanol, et on chauffe à reflux pendant 16 h.
On refroidit le milieu réactionnel, on dilue avec 90 ml de benzène et on filtre. On sépare la phase organique et on évapore le solvant sous pression réduite. On purifie le résidu par chromatographie sur colonne de gel de silice en éluant avec un mélange 95/5/0,5 de chloroforme, méthanol et ammoniaque. On obtient 0,80 g d'une huile orange que l'on fait cristalliser dans 4 ml d'éther de pétrole. On obtient ainsi 0,617 g de produit sous forme de solide blanc cassé. Point de fusion : 85-88 °C.

### Exemple 6 (Composé N°7).

4-(6-Chloropyrazin-2-yl)-1,4-diazabicyclo[3.2.2]nonane.

Dans un ballon tricol de 25 ml on introduit 0,30 g (2,0 mmoles) de 1,4-diazabicyclo[3.2.2]nonane, 0,30 g (2,0 mmoles) de 2,6-dichloropyrazine, 3 ml d'une solution aqueuse de soude à 50% et 64,4 mg (0,2 mmole) de bromure de tétrabutylammonium dans 3 ml de toluène et on chauffe le milieu réactionnel à 60 °C pendant 4 h.
On ajoute 0,30 g (2,0 mmoles) de 2,6-dichloropyrazine et 64,4 mg (0,2 mmole) de bromure de tétrabutylammonium et on continue de chauffer pendant 18 h.
On sépare la phase organique, on ajoute de l'eau et on extrait le mélange au chloroforme. On évapore le solvant sous pression réduite et on purifie le résidu obtenu par chromatographie sur colonne de gel de silice en éluant avec un mélange 95/5/0,5 de chloroforme, méthanol et ammoniaque et on fait cristalliser le produit obtenu dans 1,5 ml d'éther diisopropylique.
On obtient 0,17 g de produit.
Point de fusion : 80°C.

### Exemple 7 (Composé N°12).

Bromhydrate de 4-(6-phénylpyridazin-3-yl)-1,4-diazabicyclo_ [3.2.2]nonane (2:1).

Dans un ballon tricol de 25 ml on introduit 0,477 g (2,0 mmoles) de 4-(6-chloropyridazin-3-yl)-1,4-diazabicyclo_ [3.2.2]nonane, 0,293 g (2,4 mmoles) d'acide phénylboro_ nique, 69 mg (0,06 mmole) de tétrakis(triphénylphospino)_ palladium, 2 ml d'une solution aqueuse de carbonate de sodium 2M et 0,1 ml d'éthanol, et on chauffe le milieu réactionnel à reflux pendant 17 h.
On décante la phase organique, on la lave à l'eau, on l'évapore sous pression réduite et on purifie le résidu par chromatographie sur gel de silice en éluant avec un mélange 95/5/0,5 de chloroforme, méthanol et ammoniaque.
On obtient 0,49 g de produit sous forme d'huile que l'on reprend dans 5 ml d'éthanol et que l'on traite avec 0,7 ml d'une solution d'acide bromhydrique dans l'acide acétique 5M. On filtre le précipité, on le rince à l'éthanol puis à l'éther.
On obtient ainsi 0,551 g de de solide blanc.
Point de fusion : 295-298°C.

Le tableau qui suit illustre les structures chimiques et les propriétés physiques de quelques composés de l'invention

Dans la colonne "R₁", "3,4-OCR₂O-C₆R₃" désigne un groupe 3,4-méthylènedioxyphényle, ou 1,3-benzodioxol-5-yle et "4-C₆H₅-C₆H₄" désigne un groupe biphényl-4-yle.
Dans la colonne "Sel", "-" désigne un composé à l'état de base, "HBr" désigne un bromhydrate et "HCl" désigne un chlorhydrate ; le rapport molaire acide:base est indiqué en regard.
Dans la colonne "F (°C)", "(d)" désigne un point de fusion avec décomposition.

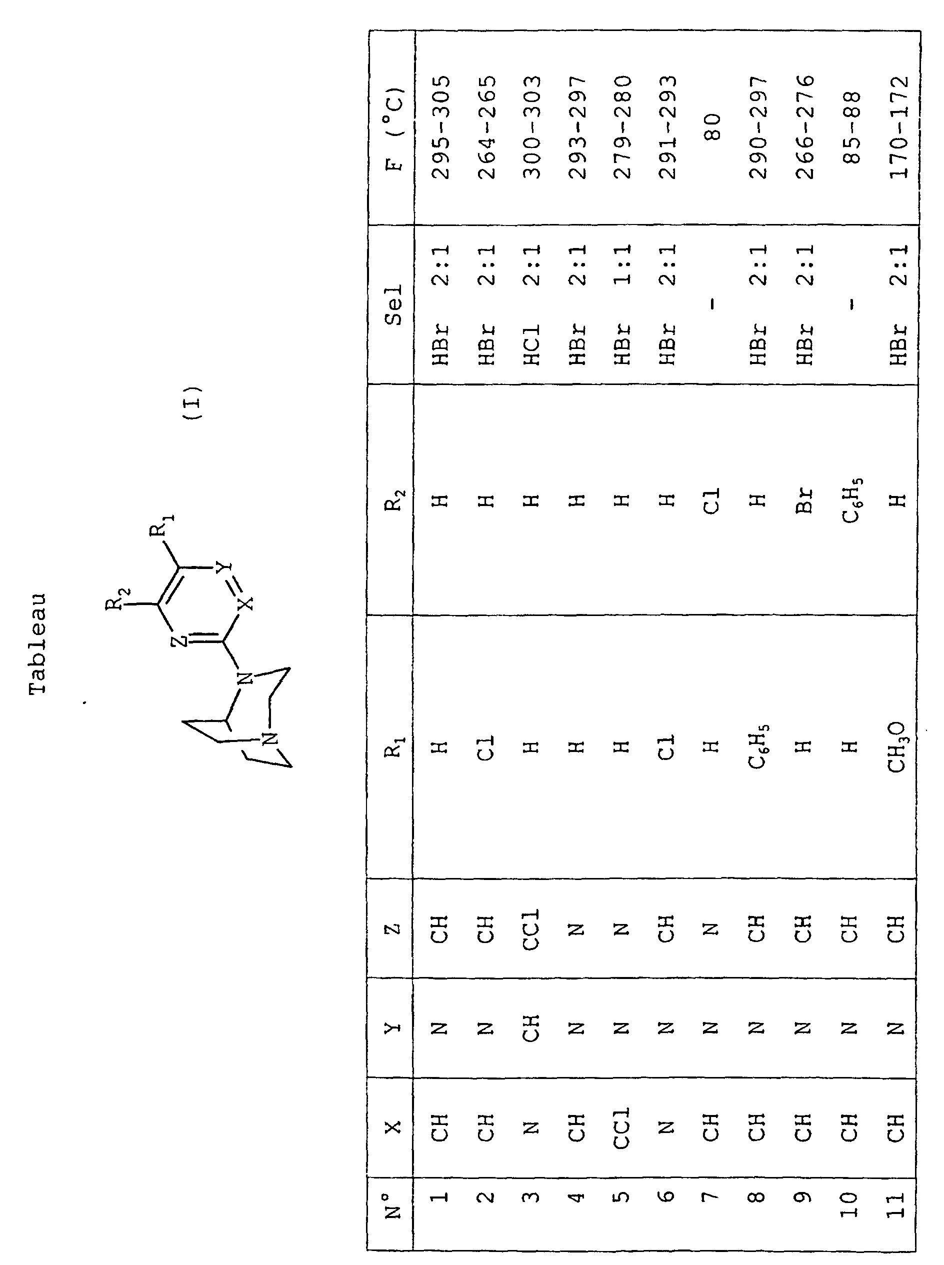

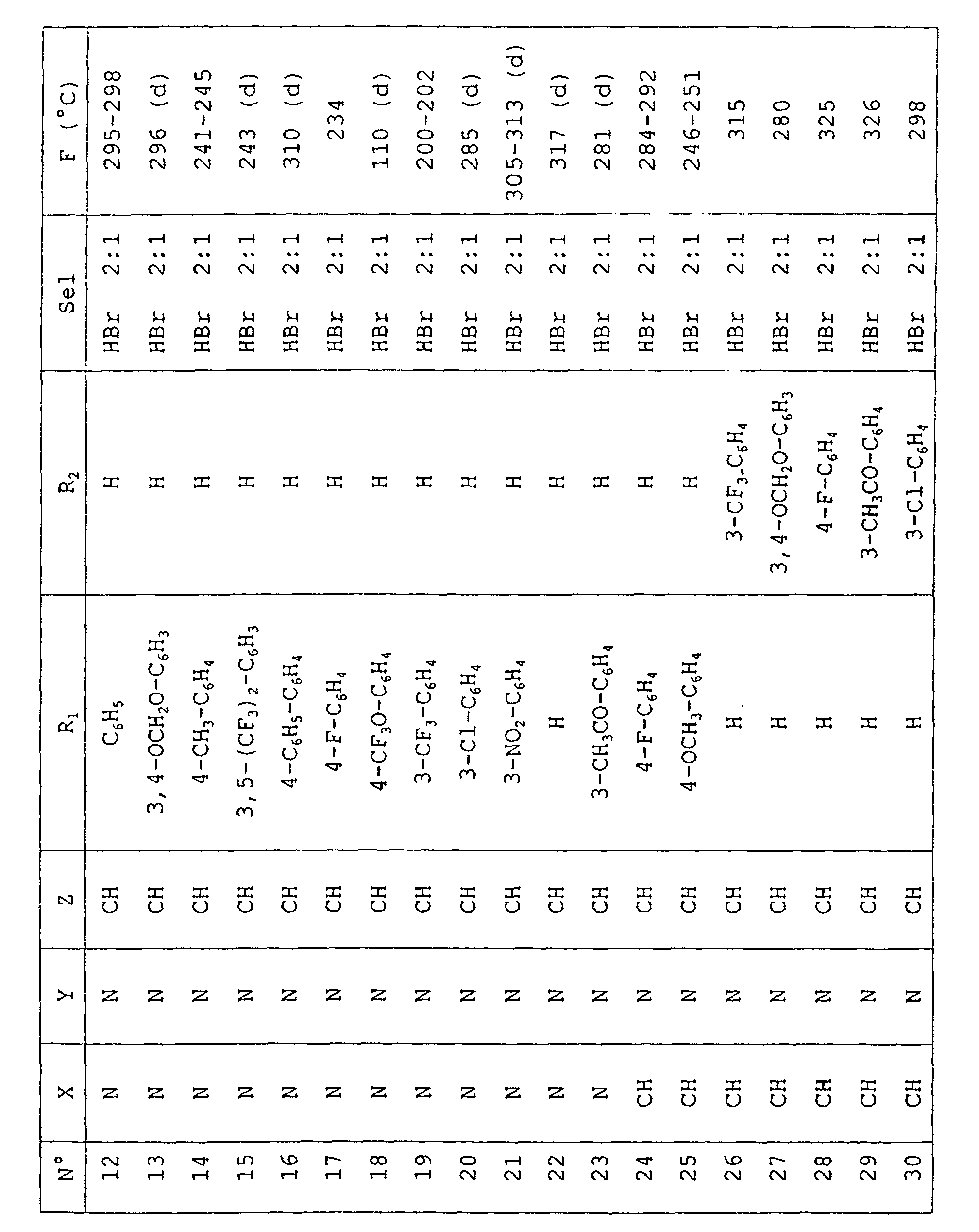

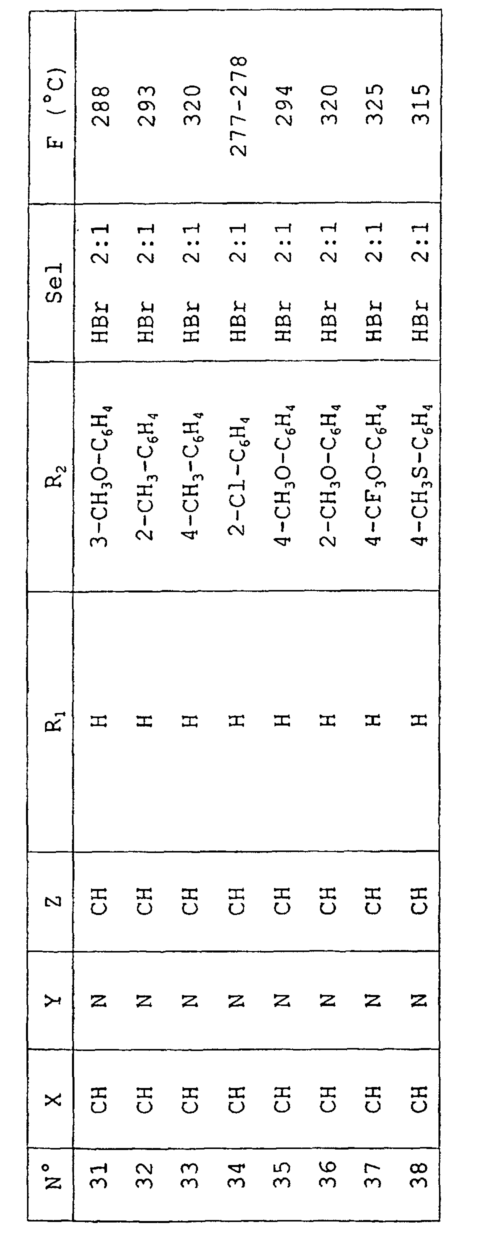

Les composés de l'invention ont fait l'objet d'essais qui ont mis en évidence leurs propriétés thérapeutiques.

Ainsi ils ont été étudiés quant à leur affinité vis-à-vis des récepteurs nicotiniques contenant la sous-unité α₄β₂ selon les méthodes décrites par Anderson et Arneric, *Eur. J. Pharmacol* (1994) **253** 261, et par Hall et coll., *Brain Res.* (1993) **600** 127.

On décapite des rats mâles Sprague Dawley de 150 à 200 g et on prélève rapidement la totalité du cerveau, on l'homogénéise dans 15 volumes d'une solution de sucrose à 0,32 M à 4°C puis on le centrifuge à 1000 g pendant 10 min. On élimine le culot, et on centrifuge le surnageant à 20000 g pendant 20 min à 4°C. On récupère le culot et on l'homogénéise à l'aide d'un broyeur Polytron™ dans 15 volumes d'eau bidistillée à 4°C, puis on le centrifuge à 8000 g pendant 20 min. On élimine le culot et on centrifuge le surnageant et la couche de peau ("buffy coat") à 40000 g pendant 20 min, on récupère le culot, on le remet en suspension dans 15 ml d'eau bidistillée à 4°C et on le centrifuge encore une fois à 40000 g avant de le conserver à -80°C.
Le jour de l'expérience on décongèle lentement le tissu et on le met en suspension dans 3 volumes de tampon. On fait incuber 150 µl de cette suspension membranaire à 4°C pendant 120 min en présence de 100 µl de [³H]cytisine 1 nM dans un volume final de 500 µl de tampon, en présence ou en absence de composé à tester. On arrête la réaction par filtration sur des filtres Whatman GF/B™ préalablement traités avec de la polyéthylèneimine, on rince les filtres avec deux fois 5 ml de tampon à 4°C, et on mesure la radioactivité retenue sur le filtre par scintigraphie liquide. On détermine la liaison non spécifique en présence de (-)-nicotine à 10 µM ; la liaison non spécifique représente 75 à 85% de la liaison totale récupérée sur le filtre. Pour chaque concentration de composé étudié on détermine le pourcentage d'inhibition de la liaison spécifique de [³H]cytisine, puis on calcule la CI₅₀, concentration de composé qui inhibe 50% de la liaison spécifique.

Les CI₅₀ des composés de l'invention les plus affins se situent entre 0,001 et 0,013 µM.

Les composés de l'invention ont aussi été étudiés quant à leur affinité vis-à-vis des récepteurs nicotiniques contenant la sous-unité α7, selon les méthodes décrites par Marks et Collins, *J.Pharmacol.Exp.Ther.* (1982) **22** 564 et Marks et al., *Mol. Pharmacol.* (1986) **30** 427.
On décapite des rats mâles OFA de 150 à 200 g, on prélève rapidement la totalité du cerveau, on l'homogénéise à l'aide d'un broyeur Polytron™ dans 15 volumes d'une solution de sucrose à 0,32 M à 4°C, puis on le centrifuge à 1000 g pendant 10 min. On élimine le culot, et on centrifuge le surnageant à 8000 g pendant 20 min à 4°C. On récupère le culot et on l'homogénéise à l'aide d'un broyeur Polytron™ dans 15 volumes d'eau bidistillée à 4°C, puis on le centifuge à 8000 g pendant 20 min. On élimine le culot et on centrifuge le surnageant et la couche de peau ("buffy coat") à 40000 g pendant 20 min. On récupère le culot, on le remet en suspension avec 15 volumes d'eau bidistillée à 4°C et on le centrifuge encore une fois à 40000 g pendant 20 min avant de le conserver à -80°C.
Le jour de l'expérience on décongèle lentement le tissu et on le met en suspension dans 5 volumes de tampon. On préincube 150 µl de cette suspension membranaire à 37°C pendant 30 min, à l'obscurité, en présence ou en absence du composé à tester. Puis le membranes sont incubées pendant 60 min à 37°C, à l'obscurité, en présence de 50 µl de [³H]α-bungarotoxine 1 nM dans un volume final de 250 µl de tampon HEPES 20 mM, polyéthylèneimine 0,05%. On arrête la réaction par filtration sur des filtres Whatman GF/C™ préalablement traités pendant 3 heures avec de la polyéthylèneimine à 0,5%. On rince les filtres avec deux fois 5 ml de tampon à 4°C, et on mesure la radioactivité retenue sur chaque filtre par scintigraphie liquide. On détermine la liaison non spécifique en présence de α-bungarotoxine à 1 µM final ; la liaison non spécifique représente environ 60% de la liaison totale récupérée sur le filtre. Pour chaque concentration de composé étudié on détermine le pourcentage d'inhibition de la liaison spécifique de [³H]α-bungarotoxine, puis on calcule la CI₅₀, concentration de composé qui inhibe 50% de la liaison spécifique.
Les CI₅₀ des composés de l'invention les plus affins se situent entre 0,002 et 0,75 µM.

Les composés de l'invention ont également été étudiés quant à leur affinité vis-à-vis des récepteurs nicotiniques périphérique de type ganglionnaire selon la méthode décrite par Houghtling et al., *Mol. Pharmacol.* (1995) **48** 280-287. La capacité d'un composé à déplacer la [³H]-epibatidine des membranes de glandes surrénales de boeuf mesure son affinité pour ce récepteur.
On décongèle des glandes surrénales de boeuf conservées à -80°C, et on les homogénéise à l'aide d'un broyeur Polytron™ dans 20 volumes de tampon Tris-HCl 50 mM à pH 7,4 à 4°C, puis on les centifuge à 35000 g pendant 10 min. On élimine le surnageant et on remet le culot en suspension dans 30 volumes de tampon Tris-HCl 50 mM à 4°C et on réhomogénéise avant de recentrifuger à 35000 g pendant 10 min. On reprend le dernier culot dans 10 volumes de tampon Tris-HCl à 4°C. On fait incuber 100 µl de membrane soit 10 mg de tissu frais à 24°C pendant 3 h en présence de 50 µl de [³H]-epibatidine 0,66 nM final dans un volume final de 250 µl de tampon, en présence ou en absence de composé à tester. On arrête la réaction par dilution des échantillons avec du tampon Tris-HCl 50 µM pH 7,4 à 4°C puis on filtre sur de filtres Whatman GF/C™ préalablement traités pendant 3 heures avec de la polyéthylèneimine à 0,5%. On rince les filtres 2 fois par 5 ml de tampon et on mesure la radioactivité retenue sur le filtre par scintigraphie liquide. On détermine la liaison non spécifique en présence de (-)nicotine 2 mM final ; la liaison non spécifique représente 30 à 40% de la liaison totale récupérée sur le filtre. Pour chaque concentration de composé étudié on détermine le pourcentage d'inhibition de la liaison spécifique de [³H]-epibatidine, puis on calcule la CI₅₀, concentration de composé qui inhibe 50% de la liaison spécifique.

Les CI₅₀ des composés de l'invention se situent entre 0,06 et 20 µM.

Les résultats des essais qui précèdent montrent que certains composés de l'invention sont des ligands sélectifs pour les sous-unités α₄β₂, α₇ ou α₃ du récepteur nicotinique et que d'autres sont mixtes α₄β₂ et α₇, α₄β₂ et α₃, ou α₇ et α₃.

Enfin les composés de l'invention ont fait l'objet d'essais qui ont mis en évidence leurs propriétés analgésiques. Ainsi ils ont été étudiés dans le modèle de la plaque chauffante, selon la méthode de Eddy et Leimbach, *J. Pharmacol. Exp. Ther.* (1953) **107** 385-393 dans le but de rechercher et quantifier un éventuel effet analgésique.
Des souris de 20 à 30 g sont soumis à un stimulus thermique par contact des pattes avec une plaque maintenue à la température constante de 57,5°C par un bain-marie thermostaté. On mesure le temps de réaction à la douleur qui se manifeste par un lèchage de pattes ou un saut. Ainsi, après le délai de prétraitement effectué par voie sous-cutanée ou orale (chaque lot étant constitué de huit animaux pour un même prétraitement), les souris sont déposées individuellement sur la plaque et le temps de réaction à la douleur est mesuré. L'animal est retiré de la plaque immédiatement après la manifestation de la douleur. Le temps maximum d'exposition au stimulus est de 30 secondes.
On exprime pour chaque lot le temps moyen de réaction accompagné de l'erreur standard à la moyenne (e.s.m). Une analyse de variance non paramétrique (Kruskal-Wallis), est effectuée sur l'ensemble du lot. Un test de Wilcoxon permet la comparaison de chaque lot traité au lot témoin. Les différences sont considérées comme statistiquement significatives au seuil 5%.
Ce temps de réaction est significativement augmenté par les analgésiques principalement à effets centraux.
Les composés de l'invention montrent une activité dans ce test aux doses comprises entre 0,3 et 30 mg/kg par voie intrapéritonéale ou orale.

Les résultats des divers essais suggèrent l'utilisation des composés dans le traitement ou la prévention des désordres liés à un dysfonctionnement des récepteurs nicotiniques, notamment au niveau du système nerveux central ou du système gastro-intestinal.

Au niveau du système nerveux central ces désordres comprennent les altérations cognitives, plus spécifiquement mnésiques, mais également attentionnelles, liées à la maladie d'Alzheimer, au vieillissement pathologique (Age Associated Memory Impairment, AAMI), au syndrome Parkinsonien, à la trisomie 21 (Down's syndrome), au syndrome alcoolique de Korsakoff, aux démences vasculaires (multi-infarct dementia, MID) et au déficit de l'attention/hyperactivité (attention deficit/hyperactivity disorder, ADHD).
Les composés de l'invention pourraient également être utiles dans le traitement des troubles moteurs observés dans la maladie de Parkinson ou d'autres maladies neurologiques telles que la chorée de Huntington, le syndrome de Tourette, la dyskinésie tardive et l'hyperkinésie.
Les composés de l'invention peuvent également constituer un traitement curatif ou symptomatique des accidents et traumatismes crâniens ou médullaires, des accidents vasculaires cérébraux et des épisodes hypoxiques cérébraux, ainsi que des autres maladies neurodégénératives aiguës ou chroniques.
Ils peuvent être utilisés dans les cas de pathologies psychiatriques : schizophrénie, dépression, anxiété, attaques de panique, comportements compulsifs et obsessionnels.
Ils peuvent prévenir les symptômes dus au sevrage au tabac, à l'alcool, aux différentes substances induisant une dépendance, telles que cocaïne, LSD, cannabis, benzodiazépines.
Enfin ils peuvent être utiles pour le traitement de la douleur aiguë et neuropathique.

Au niveau du système gastro-intestinal les composés de l'invention pourraient être utiles dans le traitement de la maladie de Crohn, de la colite ulcéreuse, du syndrome du côlon irritable et de l'obésité.

A cet effet les composés de l'invention peuvent être présentés sous toutes formes de compositions appropriées à l'administration entérale, parentérale ou transdermique, telles que comprimés, dragées, gélules, capsules, suspensions ou solutions buvables ou injectables telles que sirops ou ampoules, timbres transdermiques ("patch"), etc, associés à des excipients convenables, et dosés pour permettre une administration journalière de 0,01 à 20 mg/kg.

## Revendications

1. Composé répondant à la formule générale (I) 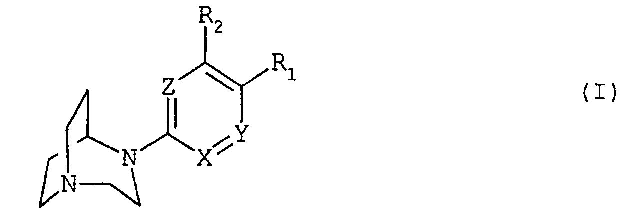 dans laquelle
l'un des symboles X, Y et Z représente un atome d'azote, un autre représente un groupe de formule C-R₃ et le troisième représente un atome d'azote ou un groupe de formule C-R₄, R₃ et R₄ représentent chacun, indépendamment l'un de l'autre, un atome d'hydrogène ou d'halogène ou un groupe trifluorométhyle, cyano, hydroxy, (C₁-C₆)alkyle ou (C₁-C₆)alcoxy,
R₁ et R₂ représentent chacun, indépendamment l'un de l'autre, un atome d'hydrogène ou d'halogène ou un groupe trifluorométhyle, cyano, hydroxy, (C₁-C₆)alkyle, (C₁-C₆)alcoxy, ou phényle éventuellement substitué par un atome d'halogène, par un ou deux groupes trifluorométhyle, par un groupe cyano, par un groupe nitro, par un groupe hydroxy, par un groupe (C₁-C₆)alkyle, par un groupe (C₁-C₆)alcoxy, par un groupe acétyle, par un groupe méthylènedioxy, par un groupe trifluorométhoxy, par un groupe méthylthio ou par un groupe phényle, à l'état de base ou de sel d'addition à un acide.

2. Composé selon la revendication 1, **caractérisé en ce que** l'hétérocycle contenant X, Y et Z est un groupe pyridin-3-yle.

3. Composé selon la revendication 1, **caractérisé en ce que** l'hétérocycle contenant X, Y et Z est un groupe pyridazin-3-yle.

4. Médicament **caractérisé en ce qu'**il consiste en un composé selon la revendication 1.

5. Composition pharmaceutique **caractérisée en ce qu'**elle contient un composé selon la revendication 1, associé à un excipient.

## Patentansprüche

1. Verbindung der allgemeinen Formel (I) 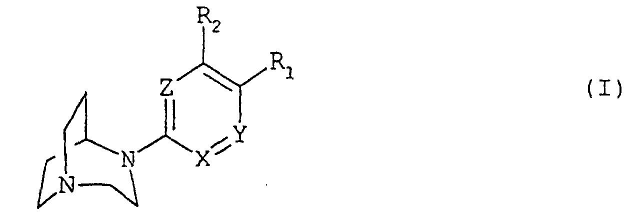 in der
eines der Symbole X, Y und Z ein Stickstoffatom, ein anderes eine Gruppe der Formel C-R₃ und das dritte ein Stickstoffatom oder eine Gruppe der Formel C-R₄ darstellen,
R₃ und R₄ jeweils unabhängig voneinander ein Wasserstoffatom, ein Halogenatom, eine Trifluormethylgruppe, eine Cyanogruppe, eine Hydroxygruppe, eine (C₁-C₆)-Alkylgruppe oder eine (C₁-C₆)-Alkoxygruppe bedeuten,
R₁ und R₂ jeweils unabhängig voneinander ein Wasserstoffatom, ein Halogenatom, eine Trifluormethylgruppe, Cyanogruppe, Hydroxygruppe, (C₁-C₆)-Alkylgruppe, (C₁-C₆)-Alkoxygruppe oder Phenylgruppe, die gegebenenfalls durch ein Halogenatom, durch eine oder zwei Trifluormethylgruppen, durch eine Cyanogruppe, durch eine Nitrogruppe, durch eine Hydroxygruppe, durch eine (C₁-C₆)-Alkylgruppe, durch eine (C₁-C₆)-Alkoxygruppe, durch eine Acetylgruppe, durch eine Methylendioxygruppe, durch eine Trifluormethoxygruppe, durch eine Methylthiogruppe oder durch eine Phenylgruppe substituiert ist, bedeuten, in Form der Base oder in Form des Additionssalzes mit einer Säure.

2. Verbindung nach Anspruch 1, **dadurch gekennzeichnet, daß** der X, Y und Z enthaltende Heterocyclus eine Pyridin-3-yl-gruppe ist.

3. Verbindung nach Anspruch 1, **dadurch gekennzeichnet, daß** der X, Y und Z enthaltende Heterocyclus eine Pyridazin-3-yl-gruppe ist.

4. Arzneimittel, **dadurch gekennzeichnet, daß** es aus einer Verbindung nach Anspruch 1 besteht.

5. Pharmazeutische Zubereitung, **dadurch gekennzeichnet, daß** sie eine Verbindung nach Anspruch 1 in Kombination mit einem Trägermaterial enthält.

## Claims

1. Compound corresponding to the general formula (I) 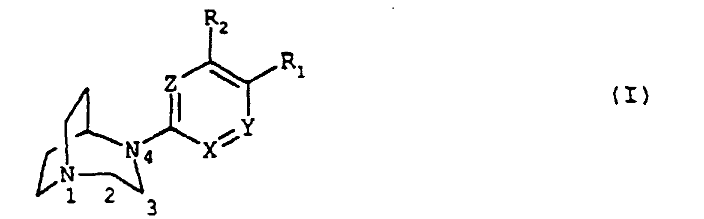 in which
one of the symbols X, Y and Z represents a nitrogen atom, another represents a group of formula C-R₃ and the third represents a nitrogen atom or a group of formula C-R₄, R₃ and R₄ represent, independently of each other, a hydrogen or halogen atom or a trifluoromethyl, cyano, hydroxyl, (C₁-C₆)alkyl or (C₁-C₆)alkoxy group,
R₁ and R₂ represent, independently of each other, a hydrogen or halogen atom or a trifluoromethyl, cyano, hydroxyl, (C₁-C₆)alkyl or (C₁-C₆)alkoxy group or a phenyl group optionally substituted with a halogen atom, with one or two trifluoromethyl groups, with a cyano group, with a nitro group, with a hydroxyl group, with a (C₁-C₆)alkyl group, with a (C₁-C₆)alkoxy group, with an acetyl group, with a methylenedioxy group, with a trifluoromethoxy group, with a methylthio group or with a phenyl group,
in the form of a base or of an addition salt with an acid.

2. Compound according to Claim 1, **characterized in that** the heterocycle containing X, Y and Z is a 3-pyridyl group.

3. Compound according to Claim 1, **characterized in that** the heterocycle containing X, Y and Z is a 3-pyridazinyl group.

4. Medicinal product, **characterized in that** it consists of a compound according to Claim 1.

5. Pharmaceutical composition, **characterized in that** it contains a compound according to Claim 1, combined with an excipient.
